# EUROPEAN PATENT APPLICATION

(11) **EP 4 843 555 A1**
(43) Date of publication of application: **25.11.2026**
(21) Application number: 25177585.4
(22) Date of filing: 20.05.2025
(51) Int. Cl.: A61K 8/44, A61K 8/46, A61Q 5/02, A61Q 19/10

(54) **COMPOSITION COMPRISING PURIFIED ALPHA-SULFO FATTY ACID SALT**

(71) Applicant: BASF SE, 67056 Ludwigshafen am Rhein (DE)
(72) Inventor: The designation of the inventor has not yet been filed
(74) Representative: BASF IP Association

(57) **Abstract**

The present invention relates to a composition comprising purified alpha-sulfo fatty acid salt (SFA), the preparation method and uses thereof as cleansing formulations.

## Description

### Field of the invention

The present invention relates to a composition comprising purified alpha-sulfo fatty acid salt (SFA), the preparation method and uses thereof as cleansing formulations.

### Background of the invention

Alpha-sulfo fatty acid salt is often used in cleansing formulations as mild alternatives to sulfate-containing surfactants for its good thickening and foaming performances. However, the thickening effect would be quite limited after introduction of additional actives and/or perfumes which are necessary ingredients for cleansing products. Besides, the thickening property of alpha-sulfo fatty acid salt is very pH dependent, and the pH for target viscosity range of many complex cleansing products would be higher than 5.0, which is not preferred for the formulations which contain organic acid preservatives such as sodium benzoate and require a pH value lower than 5.0.

Therefore, there exists a need for cleansing products comprising alpha-sulfo fatty acid salt (SFA) with a higher peak viscosity in a lower pH value.

### Summary of the invention

One object of the present invention lies in providing cleansing products comprising alpha-sulfo fatty acid salt (SFA) with a higher viscosity in a lower pH value.

It has been found that the object of the present invention can be achieved by a composition comprising a purified alpha-sulfo fatty acid salt, at least one amphoteric surfactant, at least one conditioning agent, and at least one pH adjuster, wherein the pH value of the composition is in a range of from 4.0 to 4.9 and the viscosity of the composition is in a range of from 2,000 to 40,000 mPas, and wherein the composition comprises less than 1 wt.% of sulfate and less than 1 wt.% of free fatty acid.

Particularly, the present invention relates to following aspects.

In a first aspect, the present invention provides a composition comprising a purified alpha-sulfo fatty acid salt, at least one amphoteric surfactant, at least one conditioning agent, and at least one pH adjuster, wherein the pH value of the composition is in a range of from 4.0 to 4.9 and the viscosity of the composition is in a range of from 2,000 to 40,000 mPas, and wherein the composition comprises less than 1 wt.% of sulfate and less than 1 wt.% of free fatty acid. Particularly, the composition of the present invention comprises less than 0.5 wt.% of sulfate and less than 0.5 wt.% of free fatty acid.

In a second aspect, the present invention provides a method for preparing the composition according to the present invention, comprising mixing the components of the composition.

In a third aspect, the present invention provides the use of the composition according to the invention as cleansing formulations such as shampoo, body wash or facial cleansers.

### Detailed Description of the Invention

The present invention now will be described in details hereinafter. It is to be understood that the present invention may be embodied in many different ways and shall not be construed as limited to the embodiments set forth herein. Unless mentioned otherwise, all technical and scientific terms used herein have the same meaning as commonly understood by one of ordinary skill in the art to which this invention belongs.

As used herein, the singular forms "a", "an", and "the" include plural referents unless the context clearly dictates otherwise.

As used herein, the terms "comprise", "comprising", etc. are used interchangeably with "contain", "containing", etc. and are to be interpreted in a non-limiting, open manner. That is, e.g., further components or elements may be present. The expressions "consists of" or "consists essentially of" or cognates may be embraced within "comprises" or cognates.

As used herein, "free fatty acid" refers to fatty acids that are not esterified or bound to other molecules.

The first aspect of the present invention provides a composition comprising a purified alpha-sulfo fatty acid salt, at least one amphoteric surfactant, at least one conditioning agent, and at least one pH adjuster, wherein the pH value of the composition is in a range of from 4.0 to 4.9 and the viscosity of the composition is in a range of from 2,000 to 40,000 mPas, and wherein the composition comprises less than 1 wt.% of sulfate and less than 1 wt.% of free fatty acid.

Preferably, the viscosity of the composition is in a range of from 3,000 to 35,000 mPas, more preferably from 4,000 to 32,000 mPas, most preferably from 6,000 to 30,000 mPas, for example 3,000 mPas, 4,000 mPas, 5,000 mPas, 6,000 mPas, 7,000 mPas, 8,000 mPas, 9,000 mPas, 10,000 mPas, 12,000 mPas, 14,000 mPas, 16,000 mPas, 18,000 mPas, 20,000 mPas, 22,000 mPas, 24,000 mPas, 26,000 mPas, 28,000 mPas, 30,000 mPas, 32,000 mPas, 34,000 mPas, or 35,000 mPas.

### Purified alpha-sulfo fatty acid salt

In one embodiment, the purified alpha-sulfo fatty acid salt has the formula (I)

R¹CH(SO₃M¹)COOM² (I),

in which the radical R¹ is a linear or branched alkyl or alkenyl radical with 6 to 22 carbon atoms, the radicals M¹ and M² are independently of one another selected from the group H, Li, Na, K, Ca/2, Mg/2, ammonium and alkanolamines, and the radicals M¹ and M² are not at the same time H.

In one embodiment, the radical R¹ is a linear or branched alkyl or alkenyl radical with 8 to 18 carbon atoms, preferably 10 to 16 carbon atoms, more preferably 12 to 14 carbon atoms. In a preferred embodiment, the radical R¹ is a linear or branched alkyl with 8 to 18 carbon atoms, preferably 10 to 16 carbon atoms, more preferably 12 to 14 carbon atoms. In a further preferred embodiment, the radical R¹ is a linear alkyl with 8 to 18 carbon atoms, preferably 10 to 16 carbon atoms, more preferably 12 to 14 carbon atoms.

In one embodiment, the radicals M¹ and M² are independently of one another selected from the group H, Li, Na, K, ammonium and alkanolamines, preferably Li, Na, K and ammonium, more preferably Na and K, most preferably Na. In a preferred embodiment, the alkanolamines include monoethanolamine, diethanolamine, triethanolamine or monoisopropanolamine.

In one embodiment, the purified alpha-sulfo fatty acid salt can be obtained by purifying the crude alpha-sulfo fatty acid salts according to the following steps:
(a) diluting the crude alpha-sulfo fatty acid salts in a solvent and adjusting the pH to a range of from 2 to 4;
(b) heating up the slurry of alpha-sulfo fatty acid salts obtained in the step a) until being fully solubilized;
(c) cooling down the solution of alpha-sulfo fatty acid salts obtained in the step b) to room temperature gradually to obtain the crystals;
(d) filtering the crystals obtained in the step c) to remove the mother liquor; and
(e) rinsing the filter cake at least twice with acid aqueous solution to obtain the purified alpha-sulfo fatty acid salts.

In one embodiment, the crude alpha-sulfo fatty acid salts can be prepared according to the standard process. For example, the process for preparing an alpha-sulfo fatty acid salt comprises (i) mixing solutions (in a substantially inert solvent) of the sulfur oxides and of the fatty acid in amounts such that the sulfur oxides is in molar excess (as would be expected by one skilled in the art, 1.1 to 1.5 moles of sulfur oxides per mole of fatty acid are normally appropriate); (ii) maintaining the mixture at a reaction temperature up to the boiling point of the mixture for a time sufficient for substantially complete reaction; (iii) thereafter partially neutralizing the reaction mixture by mixing it with an aqueous solution of alkali hydroxides, preferably sodium hydroxide; (iv) removing the solvent from the neutralized solution to obtain the crude alpha-sulfo fatty acid salts.

In another embodiment, the crude alpha-sulfo fatty acid salts can be commercially available, for example under Texapon^{®} SFA from BASF.

In one embodiment, the crude alpha-sulfo fatty acid salts comprise more than 4 wt.%, in particular from 6 to 14 wt.% or from 8 to 12 wt.% of sulfate (e.g. sodium sulfate) and from 0.1 to 14 wt.% of free fatty acid as impurity.

### Step (a)

The crude alpha-sulfo fatty acid salts are diluted in a solvent and the pH is adjusted to from 2 to 4, preferably 2.5 to 3.5, more preferably around 3, in particular, the pH is adjusted preferably with hydrochloric acid (e.g., with the concentration within 28 to 36 wt% as supplied commercially). The suitable solvent to dilute the crude alpha-sulfo fatty acid salts can be water or water-miscible solvent such as acetic acid, acetaldehyde, acetone, acetonitrile, butanediol, ethanol, ethylamine, ethylene glycol, formic acid, glycerol, methanol, propanol, propylene glycol, pentanediol, ethylamine etc. In some embodiment, the solvent can be acidic solution comprising one or more acids including sulfuric acid, hydrochloric acid, acetic acid, citric acid, methanesulfonic acid, p-Toluenesulfonic acid or sulfonic acid, preferably the solvent comprises water, more preferably the solvent is substantially water.

In some embodiments, the weight ratio of the alpha-sulfo fatty acid and the solvent is from 1:20 to 1:4, preferably from 1:18 to 1:5, preferably from 1:16 to 1:6, more preferably from 1:15 to 1:7, still more preferably from 1:13 to 1:8.

### Step (b) and Step (c)

A slurry of alpha-sulfo fatty acid salts is obtained in the step (a) and is then heated up until the alpha-sulfo fatty acid salts are fully solubilized (step (b), and then cooled down gradually to room temperature (step (c)) to obtain the crystals of alpha-sulfo fatty acid salts.

In some embodiments of the present invention, the solution of alpha-sulfo fatty acid salts obtained in the step (b) is cooled down to room temperature at a cooling rate of 5 to 40 minutes per degree Celsius, preferably from 5 to 30 minutes per degree Celsium. In some embodiments of the present invention, in the step (c), the solution of alpha-sulfo fatty acid salts is cooled down to from 40 to 60°C, preferably from 45 to 55 °C and this temperature is maintained for at least 1h, preferably from 1 to 2 or to 2.5 hours. The solution of alpha-sulfo fatty acid salts is then continued being cooled down to room temperature to obtain the crystals.

### Step (d)

The slurry containing crystals of the alpha-sulfo fatty acid salts obtained in the step c) are transferred into a filter press to remove the mother liquor under pressure, for example from 2 to 4 bar, to collect the crystals of the alpha-sulfo fatty acid salts.

### Step (e)

The filter cake comprising the crystals of the alpha-sulfo fatty acid salts obtained in the step (d) was rinsed with filter press operating under pressure (for example 2 to 4 bar) at least twice with acid aqueous solution to obtain the purified alpha-sulfo fatty acid salts.

In some embodiments of the present invention, the acid aqueous solution may comprise citric acid, succinic acid, oxalic acid, glycolic acid, tartaric acid, or phosphoric acid, preferably, the acid aqueous solution used in the step e) at least comprises citric acid, more preferably the acid aqueous solution comprises citric acid in an amount of 0.001% to 5%, preferably in an amount of 0.01% to 3%, still preferably in an amount of 0.03% to 2.5%, more preferably from 0.05% to 2wt%. In some embodiments of the present invention, the weight ratio of the acid aqueous solution used in the step e) to the alpha-sulfo fatty acid salts can be from 40:1 to 1:1, for example from 30:1 to 2:1, from 20:1 to 2.5:1, preferably from 15:1 to 3:1, more preferably from 11:1 to 4:1, still more preferably from 8:1 to 4.5:1, still more preferably from 7.8:1 to 5:1, most preferably from 7.8:1 to 5.6:1.

In some preferred embodiments, the filter cake is rinsed twice with acid aqueous solution and the weight ratio of the acid aqueous solution used in the two rinses is from 1:1 to 1:3.5, preferably from 1:1.5 to 1:3, more preferably from 1:1.8 to 1:2.5, preferably the acid aqueous solution comprises citric acid. In some preferred embodiments, the acid aqueous solution used in the first rinse is a solution of citric acid having pH from 2 to 4, preferably from 2.5 to 3.5, more preferably around 3 and the acid aqueous solution used in the second rinse is citric acid solution with a concentration of from 0.5 to 4 wt%, preferably 1 to 3.5 wt%, more preferably from 1.2 to 3 wt%, most preferably from 1.5 to 2.5 wt%. The purified alpha-sulfo fatty acid salts are obtained after the filtration to remove the rinsing solution.

The purified alpha-sulfo fatty acid salts contain less than 2 wt.%, preferably less than 1.5 wt.%, more preferably less than 1 wt.% of sulfate and less than 5 wt.%, preferably less than 3 wt.%, more preferably less than 2.8 wt.% of free fatty acid.

The purified alpha-sulfo fatty acid salts may be present in an amount of from 0.5 to 10 wt.%, preferably from 1 to 8 wt.%, more preferably from 2 to 6 wt.%, most preferably from 3 to 5 wt.%, based on the total weight of the composition.

### Amphoteric surfactant

In the context of the present invention, the amphoteric surfactant may be a betaine.

The betaine-type amphoteric surfactants are preferably selected from the group consisting of alkylbetaines, alkylamidoalkylbetaines, sulfobetaines, phosphobetaines, and alkylamidoalkylsulfobetaines, in particular, (C₈-C₂₄)alkylbetaines, (C₈-C₂₄)alkylamido(C₁-C₈)alkylbetaines, sulphobetaines, and (C₈-C₂₄)alkylamido(C₁-C₈)alkylsulphobetaines. In one embodiment, betaine-type amphoteric surfactants are selected from the group consisting of (C₈-C₂₄)alkylbetaines, (C₈-C₂₄)alkylamido(C₁-C₈)alkylsulphobetaines, sulphobetaines, and phosphobetaines.

Non-limiting examples that may be mentioned include the compounds classified in the CTFA dictionary, 9th edition, 2002, under the names cocobetaine, laurylbetaine, cetylbetaine, co-co/oleamidopropylbetaine, cocamidopropylbetaine, palmitamidopropylbetaine, stearamidopropylbetaine, cocamidoethylbetaine, cocamidopropylhydroxysultaine, oleamidopropylhydroxysultaine, cocohydroxysultaine, laurylhydroxysultaine, and cocosultaine, alone or as mixtures.

The betaine-type amphoteric surfactants are preferably an alkylbetaine and an alkylamidoalkylbetaine, in particular cocobetaine or cocamidopropylbetaine.

The amphoteric surfactant may be present in an amount of from 1 to 20 wt.%, preferably from 3 to 16 wt.%, more preferably from 5 to 12 wt.%, most preferably from 6 to 10 wt.%, based on the total weight of the composition.

### Conditioning agent

In the context of the present invention, the conditioning agent may be a glycerol fatty acid ester.

The conditioning agent is preferably selected from the group consisting of esters of fatty acids of chain length C₈-C₂₄, preferably C₁₀-C₂₂ fatty acids with glycerol. Preferably, the conditioning agent is a glyceryl ester of saturated or unsaturated C₈-C₂₄ fatty acid, preferably a glyceryl ester of saturated or unsaturated C₁₀-C₂₂ fatty acids, more preferably a glyceryl ester of saturated C₁₂-C₁₈ fatty acids or unsaturated C₁₆-C₂₀ fatty acids, most preferably a glyceryl ester of saturated C₁₂-C₁₆ fatty acids or unsaturated C₁₆-C₁₈ fatty acids. Suitable glyceryl esters of fatty acids for the present invention are glyceryl monooleate, glyceryl monolaurate, glyceryl monomyristate, glyceryl monopalmitate, glyceryl monopalmitoleate, glyceryl monostearate, glyceryl monohehenate, glyceryl monolinolate, glyceryl dioleate, glyceryl dilaurate, glyceryl dimyristate, glyceryl dipalmitate, glyceryl dipalmitoleate, glyceryl distearate, glyceryl dihehenate, glyceryl dilinolate and their mixture. Particularly, the most preferred glyceryl esters of fatty acids for the present invention are glyceryl monooleate and glyceryl monolaurate.

The conditioning agent may be present in an amount of from 0.01 to 10 wt.%, preferably from 0.05 to 8 wt.%, more preferably from 0.06 to 5 wt.%, most preferably from 0.08 to 2 wt.%, based on the total weight of the composition.

### pH adjuster

In the context of the present invention, the pH adjuster may include succinic acid, citric acid, sodium citrate, tartaric acid, sodium tartrate, sodium hydroxide, potassium hydroxide, triethanolamine, gluconolactone, or combinations thereof.

The pH adjuster may be present in an amount to obtain the required pH of the composition.

### Nonionic emulsifier

In one embodiment, the composition according to the present invention comprises at least one nonionic emulsifier.

In the context of the present invention, the nonionic emulsifier can be selected from the group consisting of alkyl polyglycoside, polyglyceryl fatty acid ester and their mixture.

In one embodiment of the present invention, the nonionic emulsifier may be an alkyl polyglycoside.

The alkyl polyglycoside may be at least one selected from the group consisting of C₈-C₁₀ alkyl polyglycosides, C₈-C₁₆ alkyl polyglycosides, C₁₂-C₁₆ alkyl polyglycosides, C₉-C₁₁ alkyl polyglycosides, C₁₂-C₂₀ alkyl polyglycosides, and a combination thereof. More particularly, the alkyl polyglycoside may be at least one selected from the group consisting of decyl glucoside, arachidyl glucoside, caprylyl/capryl glucoside, cetearyl glucoside, coco-glucoside, lauryl glucoside, and a combination thereof.

In an embodiment, the alkyl polyglycoside may be commercially available, for example as APG^{®}, GLUCOPON^{®}, or PLANTAREN^{®} nonionic emulsifiers from BASF, or PLANTAPON^{®} or PLANTACARE^{®} nonionic emulsifiers from BASF. Examples of such nonionic emulsifiers may include but are not limited to: GLUCOPON^{®} 225DK nonionic emulsifier - an alkyl polyglycoside in which the alkyl group contains 8 to 10 carbon atoms and having an average degree of polymerization of 1.7; GLUCOPON^{®} 425N nonionic emulsifier - an alkyl polyglycoside in which the alkyl group contains 8 to 16 carbon atoms, having an average of 10.3 carbon atoms, and having an average degree of polymerization of 1.5; GLUCOPON^{®} 625UP nonionic emulsifier - an alkyl polyglycoside in which the alkyl group contains 12 to 16 carbon atoms and having an average degree of polymerization of 1.6; APG^{®} 325N nonionic emulsifier - an alkyl polyglycoside in which the alkyl group contains 9 to 11 carbon atoms and having an average degree to polymerization of 1.5; GLUCOPON^{®} 600UP nonionic emulsifier - an alkyl polyglycoside in which the alkyl group contains 12 to 16 carbon atoms and having an average degree of polymerization of 1.4; PLANTAREN^{®} 2000 nonionic emulsifier - a C₈-C₁₆ alkyl polyglycoside in which the alkyl group contains 8 to 16 carbon atoms and having an average degree to polymerization of 1.5; PLANTAREN^{®} 1300 nonionic emulsifier - a C₁₂-C₁₆ alkyl polyglycoside in which the alkyl group contains 12 to 16 carbon atoms and having an average degree to polymerization of 1.6; GLUCOPON^{®} 220N nonionic emulsifier - an alkyl polyglycoside in which the alkyl group contains 8 to 10 carbon atoms and having an average degree of polymerization of 1.5; PLANTACARE^{®} 818 UP nonionic emulsifier - a C₈-C₁₆ fatty alcohol glucoside, Coco-glucoside (INCI); PLANTACARE^{®} 818 UP/MB* nonionic emulsifier - a C₈-C₁₆ fatty alcohol glucoside, Mass Balance, Coco-glucoside (INCI); PLANTACARE^{®} 1200 UP nonionic emulsifier - a C₁₂-C₁₆ fatty alcohol glucoside, lauryl glucoside (INCI); PLANTACARE^{®} 1200 UP/MB* nonionic emulsifier - a C₁₂-C₁₆ fatty alcohol glucoside, lauryl glucoside (INCI); PLANTACARE^{®} 2000 UP nonionic emulsifier - a C₈-C₁₆ fatty alcohol glucoside, decyl glucoside (INCI); PLANTACARE^{®} 2000 UP/MB* nonionic emulsifier - a C₈-C₁₆ fatty alcohol glucoside, Mass Balance, decyl glucoside (INCI); PLANTACARE^{®} 810 UP nonionic emulsifier - a C₈-C₁₀ fatty alcohol glucoside, caprylyl/capryl glucoside (INCI); and PLANTAPON^{®} LGC sorb nonionic emulsifier - sodium lauryl glucose carboxylate (15-25%), lauryl glucoside (10-20%) and water (60-70%), from BASF.

In one embodiment of the present invention, the nonionic emulsifier may be a polyglyceryl fatty acid ester.

In one embodiment, the polyglyceryl fatty acid ester is at least one selected from the group consisting of polyglyceryl fatty acid ester, polyglyceryl di- or tri-fatty acid ester and polyglyceryl di-polyhydroxy fatty acid ester. The polyglyceryl moiety can contain from 2 to 12, preferably from 3 to 12, more preferably from 3 to 11 glyceryl groups.

According to any one embodiment of the present invention, preferred polyglyceryl fatty acid esters are any polyglyceryl-n fatty acid esters in which the fatty acid moiety is independently derived from a saturated or olefinically unsaturated, straight or branched chain fatty acid containing from 6 to 22 carbon atoms, preferably from 8 to 18 carbon atoms, or preferably from 10 to 14 carbon atoms, such as polyglyceryl-2 caprate, polyglyceryl-4 caprate, polyglyceryl-5 caprate, polyglyceryl-10 caprate, polyglyceryl-2 caprylate, polyglyceryl-4 caprylate, polyglyceryl-5 caprylate, polyglyceryl-10 caprylate, polyglyceryl-2 laurate, polyglyceryl-4 laurate, polyglyceryl-5 laurate, polyglyceryl-9 laurate, polyglyceryl-10 laurate, polyglyceryl-11 laurate, polyglyceryl-2 myristate, polyglyceryl-4 myristate, polyglyceryl-5 myristate, polyglyceryl-9 myristate, polyglyceryl-10 myristate, polyglyceryl-11 myristate, polyglyceryl-2 oleate, polyglyceryl-4 oleate, polyglyceryl-5 oleate, polyglyceryl-9 oleate, polyglyceryl-10 oleate, polyglyceryl-11 oleate, polyglyceryl-2 stearate, polyglyceryl-4 stearate, polyglyceryl-5 stearate, polyglyceryl-9 stearate, polyglyceryl-10 stearate, polyglyceryl-11 stearate, polyglyceryl-2 isostearate, polyglyceryl-4 isostearate, polyglyceryl-5 isostearate, polyglyceryl-9 isostearate, polyglyceryl-10 isostearate or polyglyceryl-11 isostearate.

Preferred polyglyceryl di- or tri-fatty acid esters are any polyglyceryl-n di- or tri-fatty acid esters in which each fatty acid moiety is independently derived from a saturated or unsaturated, straight or branched chain fatty acid containing from 6 to 22 carbon atoms, preferably from 8 to 18 carbon atoms or preferably from 10 to 14 carbon atoms. Preferred compounds are those in which each fatty acid moiety is identical, such as polyglyceryl-5 dilaurate, polyglyceryl-9 dilaurate, polyglyceryl-10 dilaurate, polyglyceryl-11 dilaurate, polyglyceryl-5 dimyristate, polyglyceryl-9 dimyristate, polyglyceryl-10 dimyristate, polyglyceryl-11 dimyristate, polyglyceryl-5 dioleate, polyglyceryl-9 dioleate, polyglyceryl-10 dioleate, polyglyceryl-11 dioleate, polyglyceryl-5 distearate, polyglyceryl-9 distearate, polyglyceryl-10 distearate, polyglyceryl-11 distearate, polyglyceryl-9 diisostearate, polyglyceryl-10 diisostearate or polyglyceryl-11 diisostearate, polyglyceryl-9 trilaurate, polyglyceryl-10 trilaurate, polyglyceryl-11 trilaurate, polyglyceryl-5 trimyristate, polyglyceryl-9 trimyristate, polyglyceryl-10 trimyristate, polyglyceryl-11 trimyristate, polyglyceryl-5 trioleate, polyglyceryl-9 trioleate, polyglyceryl-10 trioleate, polyglyceryl-11 trioleate, polyglyceryl-5 tristearate, polyglyceryl-9 tristearate, polyglyceryl-10 tristearate, polyglyceryl-11 tristearate, polyglyceryl-5 triisostearate, polyglyceryl-9 triisostearate, polyglyceryl-10 triisostearate or polyglyceryl-11 triisostearate.

Preferred polyglyceryl di-polyhydroxy fatty acid esters are any polyglyceryl di-polyhydroxy fatty acid esters in which the fatty acid moieties are independently derived from a polyhydroxy saturated or olefinically unsaturated, straight or branched chain fatty acid containing from 6 to 22 carbon atoms, preferably from 8 to 18 carbon atoms, more preferably 10 to 14 carbon atoms.

Each polyhydroxy fatty acid moiety can contain from 2 to 8 hydroxy groups. Preferred compounds are those in which each fatty acid moiety is identical.

In one embodiment of the present invention, the composition comprises a mixture of alkyl glycoside and polyglyceryl fatty acid ester.

The nonionic emulsifier may be present in an amount of from 0.5 to 10 wt.%, preferably from 1 to 8 wt.%, more preferably from 1.5 to 5 wt.%, most preferably from 2 to 4 wt.%, based on the total weight of the composition.

### Preservative

In one embodiment, the composition according to the present invention comprises at least one preservative.

Suitable preservatives include, but are not limited to, glycerin-containing compounds (e.g., glycerin or ethylhexylglycerin or phenoxyethanol), benzyl alcohol, parabens (methylparaben, ethylparaben, propylparaben, butylparaben, isobutylparaben, etc.), sodium benzoate, ethylenediamine-tetraacetic acid (EDTA), potassium sorbate, grapefruit seed extract, benzoic acid, sorbic acid, salicylic acid, sorbic acid, lactic acid, dehydroacetic acid, citric acid and their salts, or combinations thereof. More than one preservative may be included in the composition. Other preservatives are known in the cosmetics industries and include salicylic acid, DMDM Hydantoin, formaldahyde, chlorphenism, triclosan, imidazolidinyl urea, diazolidinyl urea, sorbic acid, methylisothiazolinone, sodium dehydroacetate, dehydroacetic acid, quaternium-15, stearalkonium chloride, zinc pyrithione, sodium metabisulfite, 2-bromo-2-nitropropane, chlorhexidine digluconate, polyaminopropyl biguanide, benzalkonium chloride, sodium sulfite, sodium salicylate, citric acid, neem oil, essential oils (various), lactic acid, and vitamin e (tocopherol).

The preservative may be present in an amount of from 0.01 to 5 wt.%, preferably from 0.05 to 3 wt.%, more preferably from 0.1 to 1.5 wt.%, most preferably from 0.3 to 0.8 wt.%, based on the total weight of the composition.

In one embodiment, the compositions according to the invention further comprise the following other ingredients.

### Humectants

Suitable humectants for the compositions according to the invention are ethoxylated or non-ethoxylated mono-alcohols, diols or polyols with a low number of carbon atoms or their ethers (e.g. ethanol, isopropanol, 1,2-dipropanediol, propyleneglycol, glyerin, ethylene glycol, ethylene glycol monoethylether, ethylene glycol monobutylether (butyl glycol), propylene glycol monomethylether, propylene glycol monoethylether, propylene glycol monobutylether, diethylene glycol monomethylether; diethylene glycol monoethylether, diethylene glycol monobutylether and similar products). The polyols that come into consideration for that purpose have preferably from 2 to 15 carbon atoms and at least two hydroxy groups. The polyols may also contain further functional groups, especially amino groups, and/or may be modified with nitrogen. Typical examples are as follows: glycerol, alkylene glycols, for example ethylene glycol, diethylene glycol, propylene glycol, butylene glycol, hexylene glycol and also polyethylene glycols having an average molecular weight of from 100 to 1000 Dalton (for example Pluracare^{®} E 400 (commercially available from BASF); technical oligoglycerol mixtures having an intrinsic degree of condensation of from 1.5 to 10, for example technical diglycerol mixtures having a diglycerol content of from 40 to 50 wt%; methylol compounds, such as, especially, trimethylolethane, trimethylolpropane, trimethylolbutane, pentaerythritol and dipentaerythritol; lower alkyl-glucosides, especially those having from 1 to 8 carbon atoms in the alkyl radical, for example methyl and butyl glucoside; sugar alcohols having from 5 to 12 carbon atoms, for example sorbitol or mannitol; sugars having from 5 to 12 carbon atoms, for example glucose or saccharose; amino sugars, for example glucamine; dialcohol amines, such as diethanolamine or 2-amino-1,3-propanediol.

The humectant may be present in an amount of from 0.01 to 30 wt.%, preferably from 0.1 to 15 wt.%, more preferably from 1 to 5 wt.%, based on the total weight of the composition.

### Chelating agents

The cleansing composition of the present invention may further comprises a chelating agent. Suitable chelating agents for the compositions according to the invention may be selected from the group consisting of ethylenediamine tetraacetic acid (EDTA), dipotassium EDTA, disodium EDTA, tetrasodium EDTA, diammonium EDTA, calcium disodium EDTA, TEA-EDTA, tripotassium EDTA, trisodium EDTA, hydroxy ethyl ethylenediamine triacetic acid (HEDTA), trisodium HEDTA, and combinations thereof. In one embodiment, the chelating agent comprises disodium EDTA (for example Edeta^{®} BD (commercially available from BASF)).

The chelating agents may be present in an amount of from 0.001 to 1 wt.%, preferably from 0.01 to 1.0 wt.%, more preferably from 0.05 to 0.5 wt.%, based on the total weight of the composition.

### Thickeners

Suitable thickeners for the compositions according to the invention are polysaccharides, preferably xanthan gum (for example Rheocare^{®} XGN (commercially available from BASF)), guar gum, agar, alginates or tyloses, cellulose derivatives, for example hydroxyalkylcellulose, wherein alkyl is a C₁-C₄-alkyl, particularly hydroxyethyl-cellulose, preferably the Natrosol^{™} trademarks, especially preferably Natrosol^{™} 250 (CAS-Nr. 9004-62-0) of Herkules Incorporated, carboxymethylcellulose or hydroxycarboxymethylcellulose, starches, preferably the trademark National 465, Purity W or starch B990, and also relatively high molecular weight polyethylene glycol mono- and diesters of fatty acids, fatty alcohols, monoglycerides and fatty acids.

Suitable thickeners are also polyacrylates, crosslinked polyacrylic acids and derivatives thereof, such as Tinovis^{®} CD, and Rheocare^{®} TTA (commercially available from BASF), Carbopol^{®} (commercially available from Lubrizol), Ultrez^{®} (commercially available from Lubrizol), Luvigel^{®} EM (commercially available from BASF), Cosmedia^{®} SP (commercially available from BASF), Tinovis^{®} GTC UP (commercially available from BASF), Capigel^{®} 98 (commercially available from Seppic), Synthalene^{®} (commercially available from Sigma), the Aculyn^{®} grades from Rohm and Haas, such as Aculyn^{®} 22 (copolymer of acrylates and methacrylic acid ethoxylates with stearyl radical (20 ethylene oxide (EO) units)) and Aculyn^{®} 28 (copolymer of acrylates and methacrylic acid ethoxylates with behenyl radical (25 EO units)).

Suitable thickeners are furthermore, for example, aerosol grades (hydrophilic silicas), polyacrylamides, polyvinyl alcohol and polyvinylpyrrolidone, ethoxylated fatty acid glycerides, esters of fatty acids with polyols, such as, for example, pentaerythritol or trimethylolpropane, fatty alcohol ethoxylates with a narrowed homolog distribution or alkyl oligoglucosides, and also electrolytes such as sodium chloride and ammonium chloride.

The thickeners may be present in a conventional amount in the art and depending on their types. The person skilled in the art will be able to select the appropriate thickener and amount for the present invention.

In the context of the present invention, the thickeners may be present in an amount of from 0.01 to 3 wt.%, preferably from 0.02 to 2.0 wt.%, more preferably from 0.04 to 1.5 wt.%, based on the total weight of the composition.

### Conditioning polymers

Suitable conditioning polymers include polymeric quaternary ammonium salts, for example polymers and copolymers of dimethyl diallyl ammonium chloride, such as polyquaternium-4, polyquaternium-6, polyquaternium-7, polyquaternium-10, polyquaternium-11, polyquaternium-15, polyquaternium-16, polyquaternium-22, polyquaternium-24, polyquaternium-28, polyquaternium-32, polyquaternium-33, polyquaternium-35, polyquaternium-37, polyquaternium-39, polyquaternium-44, and the like, preferably polyquaternium-7 and polyquaternium-10.

The conditioning polymers may be present in an amount of from 0.01 to 2 wt.%, preferably from 0.03 to 1 wt.%, more preferably from 0.05 to 0.5 wt.%, most preferably from 0.08 to 0.3 wt.%, based on the total weight of the composition.

### Pearlizers

Suitable pearlizers include alkylene glycol esters, especially ethylene glycol distearate; fatty acid alkanolamides, especially coco fatty acid diethanolamide; partial glycerides, especially stearic acid monoglyceride; esters of polyvalent, unsubstituted or hydroxy-substituted carboxylic acids with fatty alcohols having from 6 to 22 carbon atoms, especially long-chain esters of tartaric acid; fatty substances, for example fatty alcohols, fatty ketones, fatty aldehydes, fatty ethers and fatty carbonates, which in total have at least 24 carbon atoms, especially laurone and distearyl ether; fatty acids, such as stearic acid, hydroxystearic acid or behenic acid, ring-opening products of olefin epoxides having from 12 to 22 carbon atoms with fatty alcohols having from 12 to 22 carbon atoms and/or polyols having from 2 to 15 carbon atoms and from 2 to 10 hydroxy groups, and mixtures of any two or more thereof.

The pearlizer may be present in an amount of from 0.05 to 5 wt%, preferably from 0.1 to 3 wt%, more preferably from 0.5 to 1.5 wt.%, most preferably from 0.8 to 1.2 wt.%, based on the total weight of the composition.

### Perfume oils

If appropriate, the compositions according to the invention can comprise perfume oils. Perfume oils which may be mentioned are, for example, mixtures of natural and synthetic fragrances. Natural fragrances are extracts from flowers (lily, lavender, rose, jasmine, neroli, Ylang-Ylang), stems and leaves (geranium, patchouli, petit grain), fruits (anis, coriander, caraway, juniper), fruit peels (bergamot, lemon, orange), roots (mace, angelica, celery, cardamom, costus, iris, calmus), woods (pinewood, sandalwood, guaiac wood, cedarwood, rosewood), herbs and grasses (tarragon, lemongrass, sage, thyme), needles and branches (spruce, fir, pine, dwarfpine), resins and balsams (galbanum, elemi, benzoe, myrrh, olibanum, opoponax). Also suitable are animal raw materials, such as, for example, civet and castoreum. Typical synthetic fragrance compounds are products of the ester, ether, aldehyde, ketone, alcohol and hydrocarbon type. Fragrance compounds of the ester type are, for example, benzyl acetate, phenoxyethyl isobutyrate, 4-tert-butyl cyclohexyl acetate, linalyl acetate, dimethylbenzylcarbinyl acetate, phenylethyl acetate, linalyl benzoate, benzyl formate, ethylmethyl phenylglycinate, allyl cyclohexylpropionate, styrallyl propionate and benzyl salicylate. The ethers include, for example, benzyl ethyl ether, the aldehydes include, for example, the linear alkanals having 8 to 18 carbon atoms, citral, citronellal, citronellyloxyacetaldehyde, cyclamenaldehyde, hydroxycitronellal, lilial and bourgeonat, the ketones include, for example, the ionones, cc-isomethylions and methyl cedryl ketone, the alcohols include anethol, citronellol, eugenol, isoeugenol, geraniol, linalool, phenylethyl alcohol and terioneol, and the hydrocarbons include primarily the terpenes and balsams. However, preference is given to using mixtures of different fragrances which together produce a pleasing scent note. Essential oils of lower volatility, which are mostly used as aroma components, are also suitable as perfume oils, for example sage oil, chamomile oil, oil of cloves, melissa oil, mint oil, cinnamon leaf oil, linden blossom oil, juniper berry oil, vetiver oil, olibanum oil, galbanum oil, labolanum oil and lavandin oil. Preference is given to using bergamot oil, dihydromyrcenol, lilial, lyral, citronellol, phenylethyl alcohol, α-hexylcinnamaldehyde, geraniol, benzyl acetone, cyclamenaldehyde, linalool, Boisambrene^{®} Forte, ambroxan, indol, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix^{®} Coeur, iso E-Super^{®}, Fixolide^{®} NP, evernyl, iraldein gamma, phenylacetic acid, geranyl acetate, benzyl acetate, rose oxide, romillat, irotyl and floramat alone or in mixtures.

The perfume oils may be present in the composition in a conventional amount.

### Active ingredients

It has been found that active ingredients of varying solubility can be homogeneously incorporated into the composition according to the invention.

According to the invention, the active ingredients can advantageously be selected from a group of NO synthase inhibitors, particularly if the composition according to the invention are to serve for the treatment and prophylaxis of the symptoms of intrinsic and/or extrinsic aging and also for the treatment and prophylaxis of the harmful effects of ultraviolet radiation on the skin. A preferred NO synthase inhibitor is nitroarginine.

Furthermore, the active ingredients are advantageously selected from a group consisting of catechins and bile acid esters of catechins and aqueous or organic extracts from plants or parts of plants which have a content of catechins or bile acid esters of catechins, such as, for example, the leaves of the plant family Theaceae, in particular of the species Camellia sinensis (green tea). Their typical ingredients (for example polyphenols or catechins, caffeine, vitamins, sugars, minerals, amino acids, lipids) are particularly advantageous.

Catechins are a group of compounds which are to be regarded as hydrogenated flavones or anthocyanidins and are derivatives of "catechins" (catechol, 3,3',4',5,7-flavanpentaol, 2-(3,4-dihydroxyphenyl)chroman-3,5,7-triol). Epicatechin ((2R,3R)-3,3',4',5,7-flavanpentaol) is also an advantageous active ingredient within the context of the present invention.

Also advantageous are plant extracts with a content of catechins, in particular extracts of green tea, such as, for example, extracts from leaves of the plants of the Camellia spec. species, very particularly of the tea varieties Camellia sinenis, C. assamica, C. taliensis and C. inawadiensis and hybrids of these with, for example, Camellia japonica.

Preferred active ingredients are also polyphenols and catechins from a group consisting of (-) catechin, (+)-catechin, (-)-catechin gallate, (-)-gallocatechin gallate, (+)-epicatechin, (-)-epicatechin, (-)-epicatechin gallate, (-)-epigallocatechin, (-)-epigallocatechin gallate.

Flavone and its derivatives (often also collectively called "flavones") are also advantageous active ingredients within the context of the present invention.

Further preferred active ingredients are sericoside, pyridoxol, vitamin K, biotin and aroma substances, bisabolol (bisabolol rac. (commercially available from BASF)), tocopherol (Covi-ox^{®} T-50 C (commercially available from BASF)), retinyl palmitate (Vitamin A-Palmitate Care (commercially available from BASF)), retinol (Retinol 50 C (commercially available from BASF)), Schizandra Chinensis Fruit Extract (Sqisandryl^{®} LS 9905 (commercially available from BASF)).

Furthermore, the active ingredients can also very advantageously be selected from a group of hydrophilic active ingredients, in particular from the following group:
α -hydroxy acids, such as lactic acid or salicylic acid and salts thereof, such as, for example, Na-lactate, Ca-lactate, TEA-lactate, urea, allantoin, serine, sorbitol, milk proteins, panthenol, chitosan, glycerin and glyceryl glucoside (for example Hydagen^{®} Aquaporin (commercially available from BASF)) or combination thereof.

The list of specified active ingredients and active ingredient combinations which can be used in the composition according to the invention is not of course intended to be limiting. The active ingredients can be used individually or in any combinations with one another.

The active ingredients may be present in the composition according to the invention in a conventional amount in the art depending on their types. The person skilled in the art will be able to select the appropriate active ingredients and amounts for the present invention.

The specified and further active ingredients which can be used in the composition according to the invention are given in DE 103 18 526 A1 on pages 12 to 17, to which reference is made at this point in its entirety.

### Additional benefit agent

The compositions of the present invention may further comprise one or more benefit agents that can provide a positive and/or beneficial effect to the substrate being cared, e.g. to the skin. The skilled person in the art is able to select according to general knowledge in the art of formulating cosmetic compositions, and the vast literature there-related, appropriate such optional ingredients for application purposes.

In one embodiment, the composition according to the invention further comprises one or more benefit agents, such as conditioners, skin conditioners, such as vitamins or their derivatives, such as vitamin B complex, including thiamine, nicotinic acid, biotin, pantothenic acid, choline, riboflavin, vitamin B6, vitamin B12, pyridoxine, inositol, carnitine, vitamins A, C, D, E, K and their derivatives, such as vitamin A palmitate, and pro-vitamins, e.g., panthenol (pro vitamin B5), panthenol triacetate and mixtures thereof; antioxidants; free-radical scavengers; abrasives, natural or synthetic; dyes; hair coloring agents; bleaching agents; hair bleaching agents; UV absorbers, such as benzophenone, bornelone, PABA (Para Amino Benzoic Acid), butyl PABA, cinnamidopropyl trimethyl ammonium chloride, disodium distyrylbiphenyl disulfonate, potassium methoxycinnamate; anti-UV agents, such as butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, octyl dimethyl PABA (padimate O), red petrolatum; antimicrobial agents; antibacterial agents, such as bacitracin, erythromycin, triclosan, neomycin, tetracycline, chlortetracycline, benzethonium chloride, phenol, parachlorometa xylenol (PCMX), triclocarban (TCC), chlorhexidine gluconate (CHG), zinc pyrithione, selenium sulfide; antifungal agents; melanin regulators; tanning accelerators; depigmenting agents, such as retinoids such as retinol, kojic acid and its derivatives such as, for example, kojic dipalmitate, hydroquinone and its derivatives such as arbutin, transexamic acid, vitamins such as niacin, vitamin C and its derivatives, azelaic acid, placertia, licorice, extracts such as chamomile and green tea, where retinol, kojic acid, and hydroquinone are preferred; skin lightening agents such as hydroquinone, catechol and its derivatives, ascorbic acid and its derivatives; skin coloring agents, such as dihydroxyacetone; liporegulators; weight-reduction agents; anti-acne agents; anti-seborrhoeic agents; anti-ageing agents; anti-wrinkle agents; keratolytic agents; anti-inflammatory agents; anti-acne agents, such as tretinoin, isotretinoin, motretinide, adapalene, tazarotene, azelaic acid, retinol, salicylic acid, benzoyl peroxide, resorcinol, antibiotics such as tetracycline and isomers thereof, erythromycin, anti-inflammatory agents such as ibuprofen, naproxen, hetprofen, botanical extracts such as alnus, arnica, artemisia capillaris, asiasarum root, calendula, chamomile, nidium, comfrey, fennel, galla rhois, hawthorn, houttuynia, hypericum, jujube, kiwi, licorice, magnolia, olive, peppermint, philodendron, salvia, sasa albomarginata, imidazoles such as ketoconazole and elubiol; refreshing agents; cicatrizing agents; vascular-protection agents; agents for the reduction of dandruff (Antidandruff agent), seborrheic dermatitis, or psoriasis, such as pyrithione salts, being formed from heavy metals such as zinc, tin, cadmium, magnesium aluminum, sodium and zirconium, like zinc pyrithione, shale oil and derivatives thereof such as sulfonated shale oil, selenium sulfide, sulfur, salicylic acid, coal tar, povidone-iodine, imidazoles such as ketoconazole, dichlorophenyl imidazolodioxalan, clotrimazole, itraconazole, miconazole, climbazole, tioconazole, sulconazole, butoconazole, fluconazole, miconazolenitrite and any possible stereo isomers and derivatives thereof such as anthralin, piroctone olamine (Octopirox), selenium sulfide, ciclopirox olamine, anti-psoriasis agents such as vitamin D analogs, e.g. calcipotriol, calcitriol, and tacaleitrol, vitamin A analogs such as esters of vitamin A, including vitamin A palmitate, retinoids, retinols, and retinoic acid, corticosteroids such as hydrocortisone, clobetasone, butyrate, clobetasol propionate; antiperspirants or deodorants, such as aluminum chlorohydrates, aluminum zirconium chlorohydrates; immunomodulators; nourishing agents; depilating agents, such as calcium thioglycolate, magnesium thioglycolate, potassium thioglycolate, strontium thioglycolate; agents for combating hair loss; reducing agents for permanent-waving; reflectants, such as mica, alumina, calcium silicate, glycol dioleate, glycol distearate, silica, sodium magnesium fluorosilicate; essential oils and fragrances.

Needless to say, the composition according to the invention should be cosmetically or dermatologically acceptable, i.e., it should contain a non-toxic physiologically acceptable medium and should be able to be applied to the skin. For the purposes of the invention, the expression "cosmetically acceptable" means a composition of pleasant appearance, odor, feel and/or taste.

The composition of the present invention can be used as leave-on and/or rinse-off personal care product, in particular rinse-off personal care products.

In a second aspect, the present invention provides a method for preparing the composition according to the invention, comprising mixing the components of the composition.

In a third aspect, the present invention provides the use of the composition according to the invention as cleansing formulations such as shampoo, body wash or facial cleansers.

It has been found that the composition comprising purified alpha-sulfo fatty acid salt according to the present invention shows unexpectedly higher peak viscosity than the composition comprising crude alpha-sulfo fatty acid salt which contains higher content of inorganic salt, fatty acid, and other byproducts or impurities, allowing formulators to have more space to introducing additional active ingredients while keeping the final viscosity falling within target range of cleansing products. Besides, it was surprisingly found that the pH value for its peak viscosity is much lower than that of the normal grade, making preserving with preservative such as sodium benzoate feasible.

### Examples

Aspects of the present invention are more fully illustrated by the following Examples, which are set forth to illustrate certain aspects of the present invention and are not to be construed as limiting thereto.

Following materials and evaluation methods were used in the Examples.

### Materials:

The materials shown in Table 1 are used.

**Table 1**

| **Ingredients** | **INCI** | **Function** |
|---|---|---|
| Demineralized Water | Aqua | |
| Purified SFA | Disodium 2-Sulfolaurate | Anionic surfactant |
| Texapon^{®} SFA (BASF) | Disodium 2-Sulfolaurate | Anionic surfactant |
| Dehyton^{®} PK 45 (BASF) | Cocamidopropyl Betaine (CAPB) | Amphoteric surfactant |
| Monomuls^{®} 90 0-18 (BASF) | Glyceryl Oleate (GMO) | Conditioning agent |
| Monomuls^{®} 90 L-12 (BASF) | Glyceryl Laurate (GML) | Conditioning agent |
| Plantacare^{®} 818 UP (BASF) | Coco-glucoside (APG) | Nonionic emulsifier |
| Emulgade^{®} Verde 10 LA (BASF) | Polyglyceryl-10 Laurate (PGLA) | Nonionic emulsifier |
| Ucare JR-400 (Dow Chemical) | Polyquaternium-10 | Conditioning Polymer |
| Salcare^{®} Super 7 AT1 (BASF) | Polyquaternium-7 | Conditioning Polymer |
| Cutina^{®} AGS (BASF) | Glycol Distearate | Pearlizer |
| Sodium Benzoate | Sodium Benzoate | Preservative |
| Citric Acid / NaOH | Citric Acid / Sodium Hydroxide | pH adjuster |

### Preparation of the purified alpha-sulfo fatty acid salts:

Crude alpha-sulfo fatty acid salts (Sulfopon^{®} SFA) were diluted in water to obtain a slurry of 700 g (alpha-sulfo fatty acid salt concentration from 8% by weight) and the pH value was adjusted to pH 3 with hydrochloric acid. The slurry was then heated up to fully solubilize the alpha-sulfo fatty acid salt and then cooled down to room temperature with the below program:
- Heating up from room temperature to 70 °C in 3.5 hours and maintaining at 70 °C until fully solubilization of the crude alpha-sulfo fatty acid salt;
- cooling down to 60 °C at the rate of 6 minutes per degree Celsium and maintaining at 60 °C for 1 hour;
- then cooling down to 50 °C at the rate of 30 minutes per degree Celsium and maintaining at 50 °C for 1 hour;
- then cooling down to 40 °C at the rate of 30 minutes per degree Celsium and maintaining at 40 °C for 1 hour;
- then cooling down to room temperature at the rate of 6 minutes per degree Celsium.

The slurry containing crystals of alpha-sulfo fatty acid salts was transferred into a filter press (SHXB-BZ-0.5L) to remove mother liquor under 2 bar for 10 min. The filter cake was rinsed and filtered as below detailed in Table 2.

**Table 2**

| **Preparation of slurry of the alpha-sulfo fatty acid salts** | | **Rinsing program and filtration** | **Filtration and rinsing time** |
|---|---|---|---|
| Concentration of the α -sulfo fatty acid salt (active matter) | pH adjuster | | |
| 8 wt.% | Hydrochloric acid | First rinsing step with 66g aqueous citric acid solution at pH of 3 | 1 hour for the first rinsing step |
| | | Second rinsing step with 134 g of 1.5 wt.% aqueous citric acid solution | 2 hours for the second rinsing step |

Composition comparison of the purified alpha-sulfo fatty acid salts and the crude alpha-sulfo fatty acid salts is showed in Table 3.

Measurement method:
Free fatty acid content in the composition: 10 mg of test sample was dissolved in 10mL methanol/water (50:50 v/v) and analysed by liquid chromatography/mass spectrometry coupling. The concentrations of components were determined by the calculation of area% distribution on the chromatography.

Sulfate content: 100 mg of test samples were weighted in independently in 100mL volumetric flasks and topped up to the mark with deionized water. The test solutions were analyzed by ion chromatography. The quantification is performed via the peak areas using external standard method.

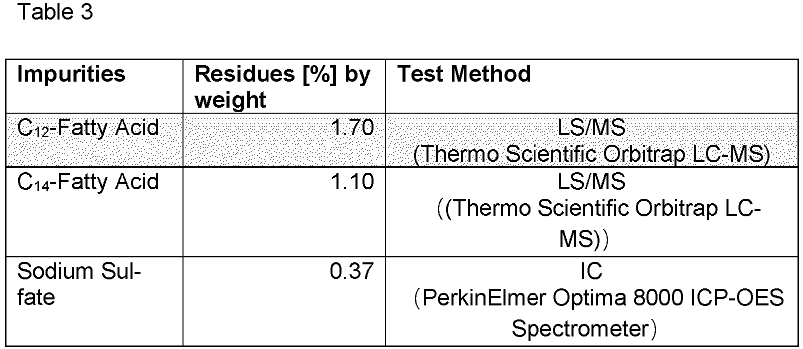

### Preparation of the compositions (200 g of batch size):

The components for preparing the compositions and the amounts thereof are shown in Table 4.
1) The SFA was added into water at room temperature and stirred at 200 rpm until an even dispersion was achieved.
2) Then amphoteric surfactant, conditioning agent and/or emulsifier were introduced and stirred at 200 rpm until all components were fully dissolved to form a clear solution or gel, at either room temperature (23°C) or 85°C based on feasibility.
3) Lastly, the preservative was added, and the pH adjuster was added to adjust the pH.

### Performance evaluation:

pH: Using a standard commercial pH meter (Mettler Toledo S210), the pH was directly measured in the compositions.

Viscosity: The viscosity was measured by Brookfield DV2T viscometer at 23°C, 4 to 12 rpm, with spindle selection of RV 02 to 06 according to viscosity range, on the next day of composition preparation.

The performance evaluations of the compositions are shown in Table 4.

**Table 4**

| Ingredients | Example 1 (wt%) | Example 2 (wt%) | Comparative Example 1 (wt%) | Example 3 (wt%) | Comparative Example 2 (wt%) | Example 4 (wt%) | Comparative Example 3 (wt%) | Example 5 (wt%) | Comparative Example 4 (wt%) | Comparative Example 5 (wt%) | Comparative Example 6 (wt%) |
|---|---|---|---|---|---|---|---|---|---|---|---|
| Demineralized Water | To 100 | To 100 | To 100 | To 100 | To 100 | To 100 | To 100 | To 100 | To 100 | To 100 | To 100 |
| Purified SFA | 4.0 | 4.0 | - | 4.0 | - | 2.4 | - | 2.4 | - | 4.0 | 4.0 |
| Texapon^{®} SFA | - | - | 4.0 | - | 4.0 | - | 2.4 | - | 2.4 | - | - |
| Dehyton^{®} PK 45 | 8.0 | 8.0 | 8.0 | 8.0 | 8.0 | 7.2 | 7.2 | 7.2 | 7.2 | 8.0 | - |
| Monomuls^{®} 90 0-18 | 1.0 | 1.0 | 1.0 | - | - | 1.0 | 1.0 | - | - | - | 1.0 |
| Monomuls^{®} 90 L-12 | - | - | - | 1.0 | 1.0 | - | - | - | - | - | - |
| Plantacare^{®} 818 UP | - | - | - | - | - | 2.4 | 2.4 | - | - | - | - |
| Emulgade^{®} Verde 10 LA | - | - | - | - | - | - | - | 2.4 | 2.4 | - | - |
| Sodium Benzoate | - | 0.50 | - | - | - | - | - | - | - | - | - |
| Citric Acid / NaOH | q.s. | q.s. | q.s. | q.s. | q.s. | q.s. | q.s. | q.s. | q.s. | q.s. | q.s. |

| Evaluation of the resulting composition | | | | | | | | | | | |
|---|---|---|---|---|---|---|---|---|---|---|---|
| Appearance | Yellowish clear gel | Yellowish clear gel | Yellow clear gel | Yellowish clear gel | Yellow clear gel | Yellowish clear gel | Yellow clear gel | Yellowish clear gel | Yellow clear gel | Yellowish clear gel | Unclear with Separation |
| Viscosity [mPas] for pH 5.5 | Not tested (< 13,900) | Not tested (<12,600) | 7,767 | Not tested (<14,800) | 3,000 | Not tested (<9,867) | 2,800 | Not tested (<7,600) | 3,033 | Not tested (<200) | Not available |
| Viscosity [mPas] for pH 5.3 | Not tested (< 13,900) | Not tested (<12,600) | 19,200 | Not tested (<14,800) | 19,700 | Not tested (<9,867) | 14,970 | Not tested (<7,600) | 13,030 | Not tested (<200) | Not available |
| Viscosity [mPas] for pH 5.1 | Not tested (<13,900) | Not tested (<12,600) | 10,670 | Not tested (<14,800) | 6,667 | Not tested (<9,867) | 11,500 | Not tested (<7,600) | 2,733 | Not tested (<200) | Not available |
| Viscosity [mPas] for pH 4.9 | 13,900 | 12,600 | 1,170 | 14,800 | 990 | 9,867 | 1,180 | 7,600 | 714 | 67 | Not available |
| Viscosity [mPas] for pH 4.5 | 27,800 | 25,000 | 396 | 28,870 | 398 | 17,770 | 574 | 14,300 | 300 | 6,067 | Not available |
| Viscosity [mPas] for pH 4.2 | 26,930 | 16,830 | Not tested (<396) | 23,300 | Not tested (<398) | 15,100 | Not tested (<574) | 9,033 | Not tested (<300) | 11,761 | Not available |
| Viscosity [mPas] for pH 4.0 | 14,830 | 8,867 | Not tested (<396) | 16,800 | Not tested (<398) | 7,133 | Not tested (<574) | 2,667 | Not tested (<300) | 14,720 | Not available |
| Viscosity [mPas] for pH 3.8 | Not tested (<14,830) | Not tested (<8,867) | Not tested (<396) | Not tested (<16,800) | Not tested (<398) | Not tested (<7,133) | Not tested (<574) | Not tested (<2,667) | Not tested (<300) | 30,270 | Not available |
| Viscosity [mPas] for pH 3.6 | Not tested (<14,830) | Not tested (<8,867) | Not tested (<396) | Not tested (<16,800) | Not tested (<398) | Not tested (<7,133) | Not tested (<574) | Not tested (<2,667) | Not tested (<300) | 28,630 | Not available |
| Viscosity Range [mPas] from pH 4.0∼4.9 | 13,900 ~ 27,800 | 8,867 ~ 25,000 | <1,170 | 14,800 ~ 28,870 | <990 | 7,133 ~ 17,770 | <1,180 | 2,667 ~ 14,300 | <714 | 67-14,720 | Not available |
| pH range for viscosity within 2,000 ~ 40,000 mPas | 4.0 to 4.9 | 4.0 to 4.9 | 5.0 to 5.5 | 4.0 to 4.9 | 5.0 to 5.5 | 4.0 to 4.9 | 5.0 to 5.5 | 4.0 to 4.9 | 5.0~5.5 | 3.6-4.5 | Not available |
| Dispersion time needed in Step (1) | 5 min | 5 min | > 30 min | Not tested | Not tested | Not tested | Not tested | Not tested | Not tested | Not tested | Not available |
| Heating process required for fully dissolving SFA in Step (2) | No (room temperature) | No (room temperature) | Yes (85°C) | No (room temperature) | Yes (85°C) | No (room temperature) | Yes (85°C) | No (room temperature) | Yes (85°C) | No (room temperature) | Yes (85°C) |

As shown in Table 4, compared with normal SFA (Comparative Example 1), the purified SFA (Example 1) was unexpectedly found to reach significantly higher viscosity in the composition containing Cocamidopropyl Betaine and Glyceryl Oleate at pH 4.0 to 4.9, which is suitable for preservatives like sodium benzoate (Example 2) or other organic acid types. Besides, the pH range of purified SFA (4.0-5.0) for the chassis to reach an adequate viscosity (2,000-40,000 mPas) for formulating cleansing gels was also broader than that of normal SFA (5.0-5.5).

The same conclusion could be drawn that compared with normal SFA (Comparative Example 2), the purified SFA (Example 3) was found to reach significantly higher viscosity in the composition containing Cocamidopropyl Betaine and Glyceryl Laurate at pH 4.0 to 4.9, indicating that purified SFA performed better viscosity build-up efficacies in the composition with Cocamidopropyl Betaine and glycerol esters at pH 4.0 to 4.9.

Compared with normal SFA (Comparative Example 3), the purified SFA (Example 4) was found to reach significantly higher viscosity in the composition containing Cocamidopropyl Betaine, glycerol esters and alkyl glucosides at pH 4.0 to 4.9, indicating that alkyl glucosides are optional to be added for the purified SFA to reach similar viscosity advantages over normal SFA.

Compared with normal SFA (Comparative Example 4), the purified SFA (Example 5) was found to reach significantly higher viscosity in the composition containing Cocamidopropyl Betaine, glycerol esters and polyglycerol esters at pH 4.0 to 4.9, indicating that polyglycerol esters are optional to be added for the purified SFA to reach similar viscosity advantages over normal SFA.

### Preparation of the formulations including shampoo (Example 6), facial cleanser (Example 7) and body wash (Example 8) (200 g of batch size):

The components for preparaing the formulations and the amounts thereof are shown in Table 5.

**Table 5**

| Ingredients | Example 6 (wt%) | Example 7 (wt%) | Example 8 (wt%) |
|---|---|---|---|
| Demineralized Water | To 100 | To 100 | To 100 |
| Purified SFA | 2.4 | 2.4 | 2.4 |
| Dehyton^{®} PK 45 | 7.2 | 7.2 | 7.2 |
| Plantacare^{®} 818 UP | 2.4 | 2.4 | 2.4 |
| Monomuls^{®} 90 O-18 | 1.0 | 1.0 | 1.0 |
| Ucare JR-400 | 0.2 | - | - |
| Salcare^{®} Super 7 AT1 | - | - | 0.1 |
| Cutina AGS | 1.0 | - | 1.0 |
| Sodium Benzoate | 0.5 | 0.5 | 0.5 |
| Citric Acid / NaOH | q.s. | q.s. | q.s. |
| Appearance | Viscous liquid with pearly shine | Clear gel | Clear gel |

The resulting formulations prepared from components shown in Table 5 are stable and homogeneous.

Although the embodiments and examples of the present disclosure are described above, the skilled person in the art should understand that they are only for illustrative purpose and are not intended to limit the protection scope of the present disclosure. The protection scope of the present disclosure is defined by the appended claims. The skilled person in the art can make various modifications, equivalent substitutions or improvements to these embodiments without departing from the scope and spirit of the present disclosure, but these modifications, equivalent substitutions or improvements fall within the protection scope of the present disclosure.

## Claims

**1.** A composition comprising a purified alpha-sulfo fatty acid salt, at least one amphoteric surfactant, at least one conditioning agent, and at least one pH adjuster, wherein the pH value of the composition is in a range of from 4.0 to 4.9 and the viscosity of the composition is in a range of from 2,000 to 40,000 mPas, and wherein the composition comprises less than 1 wt.% of sulfate and less than 1 wt.% of free fatty acid.

**2.** The composition according to claim 1, wherein the purified alpha-sulfo fatty acid salt has the formula (I)
R¹CH(SO₃M¹)COOM² (I),
in which the radical R¹ is a linear or branched alkyl or alkenyl radical with 6 to 22 carbon atoms, the radicals M¹ and M² are independently of one another selected from the group H, Li, Na, K, Ca/2, Mg/2, ammonium and alkanolamines, and the radicals M¹ and M² are not at the same time H.

**3.** The composition according to claim 1 or 2, wherein the purified alpha-sulfo fatty acid salts are present in an amount of from 0.5 to 10 wt.%, preferably from 1 to 8 wt.%, more preferably from 2 to 6 wt.%, most preferably from 3 to 5 wt.%, based on the total weight of the composition.

**4.** The composition according to any one of claims 1 to 3, wherein the amphoteric surfactant is a betaine, which is selected from the group consisting of alkylbetaines, alkylamidoalkylbetaines, sulfobetaines, phosphobetaines, and alkylamidoalkylsulfobetaines, preferably (C₈-C₂₄)alkylbetaines, (C₈-C₂₄)alkylamido(C₁-C₈)alkylbetaines, sulphobetaines, and (C₈-C₂₄)alkylamido(C₁-C₈)alkylsulphobetaines, more preferably cocobetaine or cocamidopropylbetaine.

**5.** The composition according to any one of claims 1 to 4, wherein the amphoteric surfactant is present in an amount of from 1 to 20 wt.%, preferably from 3 to 16 wt.%, more preferably from 5 to 12 wt.%, most preferably from 6 to 10 wt.%, based on the total weight of the composition.

**6.** The composition according to any one of claims 1 to 5, wherein the conditioning agent is a glycerol fatty acid ester, which is selected from the group consisting of esters of fatty acids of chain length C₈-C₂₄, preferably C₁₀-C₂₂ fatty acids with glycerol, preferably a glyceryl ester of saturated or unsaturated C₁₀-C₂₂ fatty acids, more preferably a glyceryl ester of saturated C₁₂-C₁₈ fatty acids or unsaturated C₁₆-C₂₀ fatty acids, most preferably a glyceryl ester of saturated C₁₂-C₁₆ fatty acids or unsaturated C₁₆-C₁₈ fatty acids.

**7.** The composition according to any one of claims 1 to 6, wherein the conditioning agent is present in an amount of from 0.01 to 10 wt.%, preferably from 0.05 to 8 wt.%, more preferably from 0.06 to 5 wt.%, most preferably from 0.08 to 2 wt.%, based on the total weight of the composition.

**8.** The composition according to any one of claims 1 to 7, wherein the pH adjuster includes succinic acid, citric acid, sodium citrate, tartaric acid, sodium tartrate, sodium hydroxide, potassium hydroxide, triethanolamine, gluconolactone, or combinations thereof.

**9.** The composition according to any one of claims 1 to 8, which comprises at least one nonionic emulsifier.

**10.** The composition according to claim 9, wherein the nonionic emulsifier is selected from the group consisting of alkyl polyglycoside, polyglyceryl fatty acid ester and their mixtures.

**11.** The composition according to claim 1 to 10, wherein the nonionic emulsifier is an alkyl polyglycoside, which is selected from the group consisting of C₈-C₁₀ alkyl polyglycosides, C₈-C₁₆ alkyl polyglycosides, C₁₂-C₁₆ alkyl polyglycosides, C₉-C₁₁ alkyl polyglycosides, and C₁₂-C₂₀ alkyl polyglycosides.

**12.** The composition according to any one of claims 1 to 10, wherein the nonionic emulsifier is polyglyceryl fatty acid ester, which is selected from the group consisting of polyglyceryl fatty acid ester, polyglyceryl di- or tri-fatty acid ester and polyglyceryl di-polyhydroxy fatty acid ester.

**13.** The composition according to any one of claims 1 to 12, which comprises at least one preservative, preferably, the at least one preservative is selected from the group consisting of glycerin-containing compounds, benzyl alcohol, parabens, sodium benzoate, ethylenediaminetetraacetic acid, potassium sorbate, grapefruit seed extract, benzoic acid, sorbic acid, salicylic acid, sorbic acid, lactic acid, dehydroacetic acid, citric acid and their salts, and a combination thereof.

**15.** A method for preparing the composition according to any one of claims 1 to 14, comprising mixing the components of the composition.

**16.** Use of the composition according to any one of claims 1 to 14 as cleansing formulations such as shampoo, body wash or facial cleansers.
